(19) Europäisches Patentamt
European Patent Office
Office européen des brevets

(11) **EP 2 499 971 B1**

(12) **FASCICULE DE BREVET EUROPEEN**

(45) Date de publication et mention
de la délivrance du brevet:
**30.12.2015 Bulletin 2015/53**

(51) Int Cl.:
*A61B 5/11* *(2006.01)*    *A61B 7/04* *(2006.01)*
*A61N 1/37* *(2006.01)*    *A61B 5/0402* *(2006.01)*
*A61N 1/365* *(2006.01)*

(21) Numéro de dépôt: **12153641.1**

(22) Date de dépôt: **02.02.2012**

(54) **Dispositif de traitement adaptatif d'un signal d'accélération endocardiaque**

Adaptative Datenverarbeitungsvorrichtung eines endokardialen Beschleunigungssignals

Device for adaptive processing of an endocardial acceleration signal

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **18.03.2011 FR 1152271**

(43) Date de publication de la demande:
**19.09.2012 Bulletin 2012/38**

(73) Titulaire: **Sorin CRM SAS**
**92140 Clamart (FR)**

(72) Inventeurs:
• **Baumann, Oliver**
**Bitterne**
**Southampton SO19 4BT (GB)**
• **Giorgis, Lionel**
**22000 Saint Brieuc (FR)**

(74) Mandataire: **Dupuis-Latour, Dominique et al**
**Bardehle Pagenberg**
**10, boulevard Haussmann**
**75009 Paris (FR)**

(56) Documents cités:
**EP-A1- 1 533 001    EP-A1- 2 092 885
US-A- 5 255 186    US-B1- 6 280 394**

**Description**

L'invention concerne les "dispositifs médicaux implantables actifs" tels que définis par la directive 90/385/CEE du 20 juin 1990 du Conseil des communautés européennes, plus précisément les implants permettant de surveiller en continu le rythme cardiaque et délivrer si nécessaire au coeur des impulsions électriques de stimulation, de défibrillation et/ou de resynchronisation en cas de trouble du rythme détecté par le dispositif.

Elle concerne plus particulièrement ceux de ces dispositifs qui sont pourvus d'un capteur d'accélération endocardiaque. Le signal délivré par ce capteur (ci-après "signal EA") est traité puis analysé par divers algorithmes de diagnostic, de pilotage du dispositif, de recherche de configurations de stimulation procurant la plus grande efficacité sur le plan hémodynamique, etc.

En effet, diverses études cliniques ont montré que l'accélération endocardiaque est un paramètre qui reflète très précisément et en temps réel les phénomènes liés aux contractions et relaxations du muscle cardiaque, et permet de ce fait de fournir des informations très complètes sur la mécanique cardiaque, aussi bien dans le cas d'un fonctionnement normal que d'un fonctionnement déficient.

Le signal d'accélération endocardiaque EA peut se décomposer en deux composantes successives correspondant aux deux principaux bruits cardiaques (sons S1 et S2 du phonocardiogramme) : la composante EA1, qui débute à la suite du complexe QRS, est engendrée par une combinaison de la fermeture des valves auriculo-ventriculaires, de l'ouverture des valves semi-lunaires et de la contraction du ventricule gauche ; la composante EA2 qui lui fait suite, quant à elle, accompagne la fin de la systole ventriculaire et elle est générée par la fermeture des valves semi-lunaires. Le signal EA contient parfois une ou deux autres composantes, appelées EA3 et EA4, correspondant aux sons S3 et S4 du phonocardiogramme. Le EP 2 092 885 A1 (ELA Medical) décrit un dispositif mettant en oeuvre une technique perfectionnée d'analyse du signal EA permettant d'en extraire un certain nombre d'informations significatives, représentatives de l'activité mécanique et hémodynamique du coeur du patient.

Cette technique opère un moyennage du signal et un recalage de ses composantes sur plusieurs cycles successifs, qui permet d'éliminer efficacement l'influence des variations cycle-à-cycle du signal EA, qui sont susceptibles de fausser les résultats délivrés par l'algorithme chargé d'analyser ce signal EA.

Plus précisément, cette technique consiste à effectuer un prétraitement du signal EA recueilli en continu, avec :

- découpage du signal EA en sous-signaux correspondant chacun à la durée d'un cycle cardiaque et repérés par un marqueur de début de cycle permettant de réaliser ce découpage ;
- segmentation de chacun de ces sous-signaux de manière à individualiser les composantes EA1 et EA2 dans une fenêtre temporelle donnée ;
- pour la composante courante EA1 (ou EA2) ainsi isolée sur un cycle, recherche d'un pic d'intercorrélation par rapport aux composantes EA1 (ou EA2) des autres cycles recueillis ;
- calcul d'un décalage temporel correspondant ;
- application du décalage temporel ainsi calculé à la composante courante, de manière à aligner celle-ci par rapport aux autres ; et
- moyennage des divers sous-signaux ainsi recalés, de manière à produire un signal EA moyen sur un cycle, avec élimination du biais de la variabilité cycle à cycle.

Le moyennage du signal EA sur plusieurs cycles permet de réduire l'influence des variations cycle-à-cycle de ce signal, mais il introduit une constante de temps d'autant plus importante que la moyenne est calculée sur un nombre de cycles élevé.

Le choix des paramètres de prétraitement du signal EA repose donc sur un compromis entre précision (d'autant meilleure que la moyenne est calculée sur un nombre important de cycles) et rapidité de réponse.

Ainsi, un calcul sur un nombre de cycles relativement faible, typiquement cinq cycles comme cela est décrit dans le document précité, permet un suivi pratiquement en temps réel de l'évolution du signal EA, mais au prix d'une certaine contamination par du bruit dû à l'instabilité du signal EA, et donc reflétant moins bien l'activité mécanique et hémodynamique du coeur du patient. De même, les critères d'admission ou de rejet d'un cycle donné pour le calcul de la moyenne seront plus ou moins rigoureux selon que le cycle de référence est calculé sur un nombre élevé de cycles élémentaires, avec par voie de conséquence un risque plus ou moins grand d'introduire des cycles atypiques (par exemple des extrasystoles) dans le calcul de la moyenne.

Le but de l'invention est de proposer une technique de traitement du signal EA qui permette de dépasser ce compromis, grâce à une adaptation dynamique des conditions de calcul du signal moyenné.

Plus précisément, l'invention propose un dispositif comprenant, comme divulgué par le EP 2 092 885 A1 précité : des moyens de recueil en continu d'un signal EA sur une séquence de N cycles cardiaques ; des moyens de découpage du signal EA recueilli, en sous-signaux EA chacun sur la durée d'un cycle cardiaque ; des moyens séparateurs, aptes à isoler dans chacun des sous-signaux EA, sur la durée d'une fenêtre d'analyse respective, au moins une composante EAx associée à l'un des bruits cardiaques S1, S2, S3 ou S4 pour ce sous-signal ; des moyens pour opérer une intercorrélation entre les sous-signaux EA de ladite au moins une composante EAx, aptes à définir un cycle de référence, déterminer pour chaque cycle le coefficient de corrélation et le décalage temporel de la composante EAx par rapport au cycle de référence, et opérer un recalage temporel relatif de la composante EAx par rapport au cycle de référence ; des moyens de sélection, aptes à appliquer aux sous-signaux EA et aux composantes EAx associées de la séquence de N cycles cardiaques une série de critères de validation et ne retenir que les N cycles répondant à ces critères, avec $N' \leq N;$ et des moyens de moyennage des N cycles cardiaques, aptes à délivrer en sortie un signal EA global moyen sur un cycle. Le dispositif comprend en outre des moyens de détection, aptes à détecter un changement d'état du patient ou la survenue d'un événement prédéterminé chez le patient, et des moyens d'adaptation dynamique des moyens de traitement, aptes à modifier, sur détection dudit changement d'état ou dudit événement prédéterminé, au moins l'un desdits critères de validation et/ou au moins l'un des paramètres des moyens de traitement.

De façon caractéristique, selon l'invention, les moyens d'adaptation dynamique des moyens de traitement sont des moyens aptes notamment à modifier au moins l'un des paramètres suivants : nombre N de cycles de la séquence recueillie et traitée ; et pas séparant deux séquences recueillies et traitées successives.

De plus, ils sont aptes à modifier au moins l'un des critères de validation suivants : seuil minimal pour ledit coefficient de corrélation par rapport au cycle de référence ; seuil maximal pour ledit décalage temporel par rapport au cycle de référence ; nombre minimal de cycles pour les N cycles retenus par les moyens de sélection ; et, en cas de pluralité de composantes EAx isolées dans chacun des sous-signaux EA, sélection des seuls sous-signaux EA dont toutes les composantes EAx associées répondent aux critères de validation.

Ledit changement d'état ou événement prédéterminé peut être choisi dans le groupe formé par : augmentation du rythme cardiaque, instabilité du rythme cardiaque, diminution de l'amplitude du pic d'accélération endocardiaque, augmentation du rythme ventilatoire, détection d'apnée.

On va maintenant décrire un exemple de mise en oeuvre de l'invention, en référence aux dessins annexés où les mêmes références numériques désignent d'une figure à l'autre des éléments identiques ou fonctionnellement semblables.

La Figure 1 est une série de trois chronogrammes illustrant différents signaux caractérisant l'activité cardiaque au cours d'un cycle donné.

La Figure 2 illustre le découpage d'un enregistrement continu en une série de cycles cardiaques successifs.

La Figure 3 est un organigramme général des différentes étapes du traitement de moyennage du signal EA en vue de son analyse ultérieure.

La Figure 4 illustre l'extraction des deux composantes EA1 et EA2 sur un sous-signal EA.

La Figure 5 illustre un exemple de calcul de fonctions d'intercorrélation entre deux sous-signaux, pour recaler les composantes des différents cycles successifs.

La Figure 6 illustre la génération du cycle moyen à partir des données prétraitées pour chacune des deux composantes EA1 et EA2.

La Figure 7 est un organigramme schématique des principales étapes de mise en oeuvre de l'algorithme de sélection des paramètres selon l'invention.

La Figure 8 est homologue de la Figure 7, pour un exemple pratique consistant à adapter les paramètres du traitement en fonction de la détection ou non d'une tachycardie ventriculaire.

On va maintenant décrire un exemple de réalisation du dispositif de l'invention.

En ce qui concerne ses aspects logiciels, l'invention peut être mise en oeuvre par une programmation appropriée du logiciel de commande d'un stimulateur connu, par exemple de type stimulateur cardiaque, resynchroniseur et/ou défibrillateur, comprenant des moyens d'acquisition d'un signal fourni par des sondes endocavitaires et/ou un ou plusieurs capteurs implantés.

L'invention peut notamment être appliquée aux dispositifs implantables tels que ceux des familles *Paradym* et *Ovatio* produits et commercialisés par Sorin CRM, Clamart, France.

Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

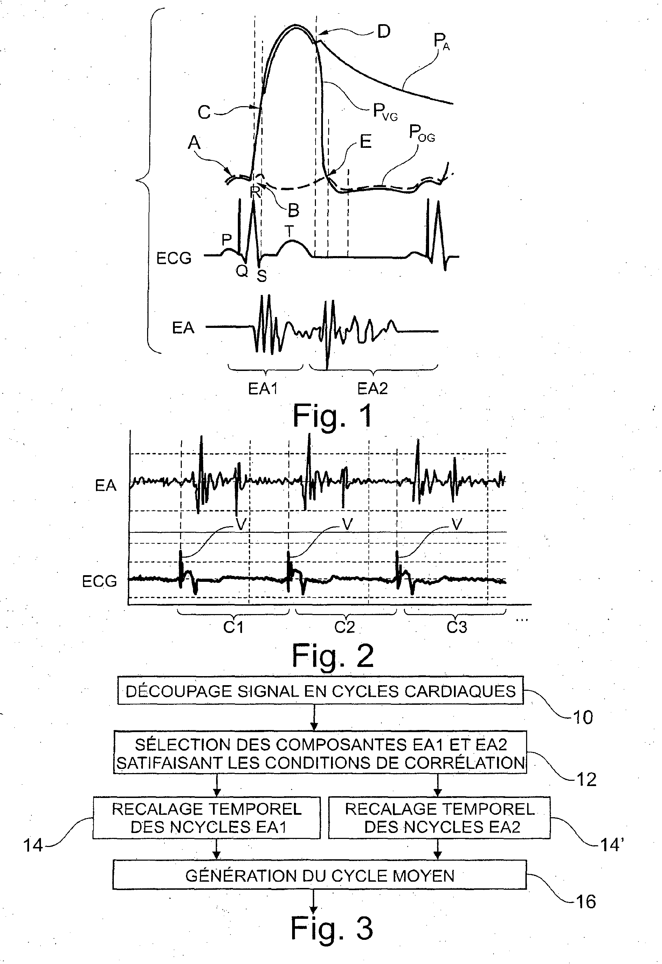

La Figure 1 illustre les différents signaux caractérisant l'activité du coeur au cours d'un cycle cardiaque, avec : le profil des pressions intracardiaques ($P_A$, $P_{VG}$ et $P_{OG}$), un relevé d'électrocardiogramme de surface (ECG), et les variations du signal d'accélération endocardiaque (EA).

La caractéristique $P_A$ illustre les variations de la pression aortique, $P_{VG}$ celles du ventricule gauche et $P_{OG}$ celles dans l'oreillette gauche. Les points A à E correspondent aux différentes phases suivantes : A contraction de l'oreillette gauche, B fermeture de la valvule mitrale, C ouverture de la valvule aortique, D fermeture de la valvule aortique, E ouverture de la valvule mitrale.

Le signal ECG présente successivement l'onde P correspondant à la dépolarisation des oreillettes, le complexe QRS correspondant à la dépolarisation des ventricules et l'onde T de repolarisation ventriculaire. L'accélération endocardiaque EA peut être mesurée par un accéléromètre directement en contact avec le muscle cardiaque (généralement à l'apex ventriculaire droit, parfois dans l'oreillette droite, ou encore contre le septum). Le EP 0 515 319 A1 (Sorin Biomedica Cardio SpA) enseigne ainsi la manière de recueillir un signal d'accélération endocardiaque au moyen d'une sonde endocavitaire pourvue d'une électrode distale de stimulation implantée au fond du ventricule et intégrant un microaccéléromètre permettant de mesurer l'accélération endocardiaque.

Le signal d'accélération endocardiaque recueilli au cours d'un cycle cardiaque forme deux composantes principales, correspondant aux deux bruits majeurs du coeur (sons S1 et S2 du phonocardiogramme) qu'il est possible de reconnaître dans chaque cycle cardiaque :

- la première composante d'accélération endocardiaque ("EA1"), dont les variations d'amplitude sont étroitement liées aux variations de la pression dans le ventricule (l'amplitude maximale crête-à-crête de cette composante EA1, appelée PEA1, étant plus précisément corrélée au maximum positif de la variation de pression dP/dt dans le ventricule gauche) et peuvent donc constituer un paramètre représentatif de la contractilité du myocarde, elle-même liée au niveau d'activité du système sympathique ;
- la seconde composante d'accélération endocardiaque ("EA2") qui, quant à elle, survient pendant la phase de relaxation ventriculaire isovolumique. Cette seconde composante est produite, principalement, par la fermeture des valves aortiques et pulmonaires.

Le signal EA contient parfois une ou deux autres composantes, appelées EA3 et EA4, correspondant aux sons S3 et S4 du phonocardiogramme et qui témoignent le plus souvent d'une insuffisance cardiaque (EA3 étant lié aux vibrations des parois du myocarde pendant le remplissage rapide, et EA4 à la contraction auriculaire).

Le terme "composante EAx" se référera par la suite à l'une de ces quatre composantes, en particulier pour désigner indifféremment la composante EA1 ou la composante EA2.

Compte tenu de la variabilité cycle-à-cycle relativement importante du signal EA, l'analyse de ce signal implique généralement de procéder à un moyennage sur plusieurs cycles, de manière à disposer d'un signal EA global moyen, défini sur la durée d'un cycle, qui sera représentatif de l'activité hémodynamique du coeur du patient et pourra ensuite être appliqué à divers algorithmes d'analyse, de diagnostic, etc.

L'invention vise plus particulièrement cette étape de traitement préalable consistant à moyenner le signal EA sur plusieurs cycles.

Le signal EA étant recueilli en continu, il importe tout d'abord d'individualiser les cycles successifs.

A cet effet, comme illustré Figure 2, les cycles cardiaques successifs C1, C2, C3 ... du signal EA continu sont définis par des marqueurs de début de cycle permettant d'individualiser ces cycles cardiaques, de manière à produire une série de sous-signaux EA correspondant chacun à une durée d'un seul cycle cardiaque. Ces marqueurs temporels de début de cycle peuvent être fournis par l'implant lui-même, qui garde en mémoire les instants de stimulation V (comme illustré Figure 2) ou les instants de détection de l'onde R, selon le mode de fonctionnement, ou encore être fournis par un algorithme de détection des pics de stimulation ou des complexes QRS du signal ECG.

Cette étape peut également mettre en oeuvre un algorithme de détection d'extrasystoles, en lui-même connu, permettant d'éliminer les cycles cardiaques affectés par les extrasystoles détectées, à savoir : le cycle précédant l'extrasystole, le cycle incluant l'extrasystole elle-même, ainsi que le cycle suivant l'extrasystole. Le signal EA à traiter sera donc le signal recueilli en continu par le capteur, abstraction fait de ces cycles non significatifs.

Le traitement conduisant au signal EA moyenné est explicité par l'organigramme de la Figure 3.

La première étape du processus, référencée 10 sur la Figure 3, consiste à individualiser, comme expliqué plus haut, un nombre N de cycles cardiaques successifs par découpage du signal EA recueilli en continu et préalablement filtré par un filtre passe-bande. D'autre part, on désignera par *step* le paramètre correspondant au pas de décalage entre deux groupes de N cycles, correspondant à la génération de deux signaux EA globaux moyens successifs.

L'analyse du signal EA peut être opérée sur une fenêtre d'analyse glissante (donc avec chevauchement entre deux ensembles de *N* cycles successivement traités) de N battements, typiquement N = 5 à 20 cycles, pour suivre l'évolution temporelle des différentes caractéristiques du signal EA avec un pas de suivi correspondant au paramètre *step,* par exemple *step* = 5 cycles.

Les cycles individualisés sont normalisés par calcul d'une longueur de cycle moyenne, ou de préférence médiane, des N battements successifs, et réajustement sur cette longueur médiane commune $RR_{med}$ des fractions individualisées du signal EA, de façon à obtenir un signal avec un nombre identique d'échantillons pour chaque cycle individualisé.

Après cet ajustement, on obtient ainsi une matrice composée de N sous-signaux EA, ayant tous une même durée (égale à la durée médiane $RR_{med}$ calculée).

L'étape suivante, correspondant au bloc 12 de la Figure 3, consiste à isoler dans chaque sous-signal EA les deux composantes EA1 et EA2, de manière à pouvoir ensuite effectuer un certain nombre de traitements de manière distincte pour chacune de ces composantes EA1 et EA2. L'extraction des deux composantes EA1 et EA2 est opérée par une technique de corrélation appliquée aux N sous-signaux EA.

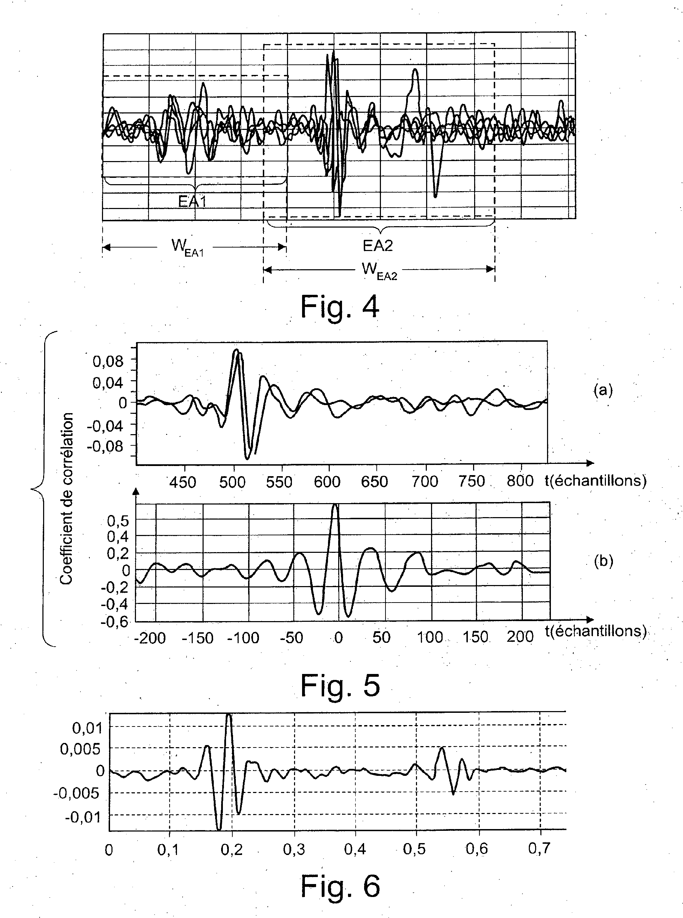

La Figure 4 illustre les deux fenêtres de signal utile EA1 et EA2 obtenues en exploitant la reproductibilité des deux composantes EA1 et EA2 sur les N sous-signaux EA. La durée de chacune de ces deux fenêtres est une valeur paramétrable $W_{EA1}$, $W_{EA2}$. On notera que les deux fenêtres de signal utile EA1 et EA2 peuvent être partiellement chevauchantes.

À partir de la matrice des N sous-signaux EA, on obtient ainsi deux matrices indépendantes contenant les échantillons respectifs $ea\_cycles_1(t)$ et $ea\_cycles_2(t)$ des composantes EA1 et EA2 des N cycles cardiaques analysés.

L'étape suivante, référencée 14 ou 14' sur la Figure 3, consiste à opérer un recalage temporel des N cycles, distinctement et parallèlement pour chacune des deux composantes EA1 et EA2 (le même traitement étant opéré pour chacune des deux composantes).

La technique utilisée consiste par exemple, pour chaque paire de sous-signaux de la matrice EA1 ou EA2, à rechercher le maximum de la fonction d'intercorrélation normalisée, fonction qui variera entre 1 (dans le cas de deux vecteurs parfaitement corrélés) et 0 (dans le cas de deux vecteurs non corrélés) :

$$\Gamma_{i,j}(\tau) = \frac{\sum_{t=0}^{Nsamples-\tau-1} \left(ea\_cycles_i(t+\tau) - \mu_{ea\_cycles_i}\right) \cdot \left(ea\_cycles_j(t) - \mu_{ea\_cycles_j}\right)}{\sqrt{\sum_{t=0}^{Nsamples-1} \left(ea\_cycles_i(t) - \mu_{ea\_cycles_i}\right)^2 \cdot \sum_{t=0}^{Nsamples-1} \left(ea\_cycles_j(t) - \mu_{ea\_cycles_j}\right)^2}}$$

Le résultat est illustré Figure 5 : la Figure 5a illustre la paire de sous-signaux analysés et la Figure 5b la fonction d'intercorrélation correspondante.

Dans l'exemple de la Figure 5, le maximum de la fonction d'intercorrélation se situe à $\tau_{i,j}$ = -5 échantillons, avec une valeur de pic de 0,78 : cela signifie qu'en retardant $ea\_cycles_1(t)$ de 5 échantillons, $ea\_cycles_1(t)$ et $ea\_cycles_2(t)$ sont corrélés avec $r$ = 0,78.

On construit alors deux tableaux :

- l'un contenant des coefficients de corrélation $r_{i,j}$ des différentes paires,
- l'autre contenant les délais $\tau_{i,j}$ permettant de recaler les différents sous-signaux $ea\_cycles_i(t)$ les uns par rapport aux autres.

On repère ensuite quel est celui des cycles le mieux corrélé aux autres $ea\_cycles_i(t)$ $(i \neq j)$, en calculant pour chaque i le coefficient de corrélation moyen avec les autres sous-signaux.

Le sous-signal présentant le coefficient de corrélation moyen maximum sera le sous-signal de référence $reference\_cycle\_ind\_EAx$.

Parmi les sous-signaux $ea\_cycles_j(t)$ (avec $j \neq reference\_cycle\_ind\_EAx$) ainsi obtenus, on ne retient que ceux qui répondent aux critères suivants :

$$r_{\text{reference\_cycle\_ind\_EAx},j} > Seuil\ de\ corrélation\_EA_x,$$

et

$$\left| \tau_{reference\_cycle\_ind\_EAx,j} \right| < Seuil\ de\ délai\ de\ corrélation\_EA_x.$$

Les deux paramètres *Seuil de corrélation_EAx* et *Seuil de délai de corrélation_EA$_x$* permettent d'éliminer du calcul ultérieur de la moyenne un certain nombre de cycles atypiques, qui risqueraient de fausser le résultat et conduire à des valeurs de moyenne "bruitées" et de ce fait difficilement exploitables.

Sur les N sous-signaux d'origine, il reste ainsi après cette étape de sélection $N'_{EAx}$ cycles EA$_x$.

À ce stade, on vérifie si le nombre $N'_{EAx}$ de cycles EAx corrects est au moins égal à un nombre minimal de cycles *N'EAx_min* pour chacune des deux composantes EA1 ou EA2.

Si cette condition n'est pas vérifiée, c'est-à-dire si l'une des deux composantes EA1 ou EA2 n'a pas satisfait le condition $N'_{EAx} > N'EAx\_min$, ceci signifie que le cycle moyen que l'on obtiendrait ne serait pas significatif ; on choisit alors de n'extraire aucune caractéristique des composantes EAx retenues, qui sont en nombre insuffisant.

En gardant uniquement les indices *j* des cycles sélectionnés, on définit les valeurs suivantes (avec $j \neq$ *reference_cycle_ind_EA$_x$*):

$$EAx\_coeff\_correl = \text{moyenne} \left( r_{\text{reference\_cycle\_ind\_EAx},j} \right),$$

et

$$EAx\_délai\_correl = \text{moyenne} \left( \tau_{\text{reference\_cycle\_ind\_EAx},j} \right)$$

L'étape suivante, correspondant au bloc 16 de la Figure 3, consiste à générer un cycle moyen à partir des composantes EA1 et EA2 prétraitées séparément de la manière indiquée plus haut.

À cet effet, les sous-signaux EA1 et EA2 de chaque cycle sont recalés par rapport au sous-signal de référence *reference_cycle_ind_EA$_x$*. On commence par centrer les valeurs $\tau_{reference\_cycle\_ind\_EAx,j}$ calculées à l'étape précédente en soustrayant la valeur moyenne des $\tau_{reference\_cycle\_ind\_EAx,j}$ sur l'ensemble des *j* (y compris le cycle de référence). On utilise ensuite ces nouvelles valeurs de $\tau_{reference\_cycle\_ind\_EAx,j}$ pour recaler les sous-signaux EA1 et EA2 par rapport au sous-signal de référence *reference_cycle_ind_EAx.* Une fois les sous-signaux recalés, il suffit de calculer une composante EA1 ou EA2 moyenne et de former ainsi le signal EA moyen par combinaison des deux composantes.

Ce signal EA global moyen, défini sur une durée d'un cycle, est illustré Figure 6.

Le traitement que l'on vient de décrire, permettant de générer un cycle global moyen du signal EA, est exposé dans le EP 2 092 885 A1 précité, auquel on pourra se référer pour plus de détails.

Comme on l'a indiqué en introduction, la mise en oeuvre de ce traitement implique un certain nombre de compromis entre précision et rapidité de la réponse, et ces compromis peuvent conduire à un signal EA global moyen reflétant mal de faibles variations de l'état hémodynamique du patient, ou bien ne révélant des variations subites importantes qu'avec un retard ne permettant pas d'exploiter de manière efficace le signal.

L'objet de la présente invention est d'améliorer ce traitement, en permettant une adaptation dynamique des divers paramètres et des critères de sélection ou d'élimination des cycles non significatifs.

Les paramètres modifiables sont notamment :

- le nombre N des cycles faisant l'objet du traitement,
- le pas *step* séparant deux séquences de traitement (avec éventuellement chevauchement).

Les critères de validation modifiables sont notamment :

- le seuil minimal du coefficient de corrélation par rapport au cycle de référence,
- le seuil minimal du décalage temporel par rapport au cycle de référence,
- le nombre minimal *N'EAx_min* de cycles subsistant après sélection,
- un indicateur précisant s'il y a lieu de rejeter un cycle dès qu'un seul des sous-signaux EA1 ou EA2 ne répond pas aux critères de validation, ou bien si le cycle ne sera accepté que si les deux sous-signaux EA1 et EA2 répondent tous deux aux critères de validation.

L'adaptation de ces paramètres et critères de validation en fonction de l'état courant du patient permet d'optimiser le fonctionnement et la réactivité du traitement du signal EA en bénéficiant :

- d'une diminution du temps de réponse pour l'obtention du signal EA global moyen, lorsqu'un résultat rapide est nécessaire, et
- de l'augmentation de la précision du signal EA global moyen obtenu lorsque le coeur n'est pas dans un état stationnaire, notamment pendant les périodes de rythme cardiaque instable.

Les événements ou changements d'état qui peuvent être pris en compte pour modifier les paramètres ou critères de validation du traitement peuvent être notamment :

- une évolution particulière de l'un des facteurs du signal EA (par exemple une chute de l'amplitude PEA1 crête-à-crête de la composante EA1), ou bien d'un signal MV (hyperventilation par exemple), ou encore d'un signal d'activité du patient (effort) ;
- une suspicion ou une détection d'arythmie ;
- une détection d'apnée ou d'hypopnée ;
- une accélération du rythme cardiaque.

L'augmentation de précision peut également être choisie dans les situations où il n'existe aucune urgence, par exemple pendant les phases de sommeil du patient.

Il peut être également souhaitable d'adapter le traitement pour limiter la charge de calcul du processeur lorsque cela n'est pas nécessaire, et préserver ainsi la durée de vie de la batterie de l'implant. Le choix d'un pas *step* plus élevé permet en particulier de réduire la charge de calcul, au prix d'un rafraichissement moins fréquent de la valeur du signal EA global moyen.

En ce qui concerne les critères de validation, si l'on choisit des critères stricts (seuil de coefficient de corrélation élevé, seuil de décalage temporel faible, nombre minimal de cycles corrects élevé), ces modifications conduiront à un taux de rejet des cycles plus important, avec une meilleure qualité finale du signal EA global moyen, mais avec le risque de, fréquemment, n'obtenir aucun signal EA global moyen, si les critères sont trop stricts.

En ce qui concerne le nombre N de cycles sur lequel est opéré le traitement de moyennage, un nombre élevé augmentera le temps nécessaire pour obtenir le signal EA global moyen, mais avec une meilleure qualité de résultat, notamment dans le cas d'un signal cyclostationnaire. Inversement, une diminution du nombre N de cycles permettra d'obtenir un résultat beaucoup plus rapidement, et sera plus adapté au cas d'un signal non cyclostationnaire.

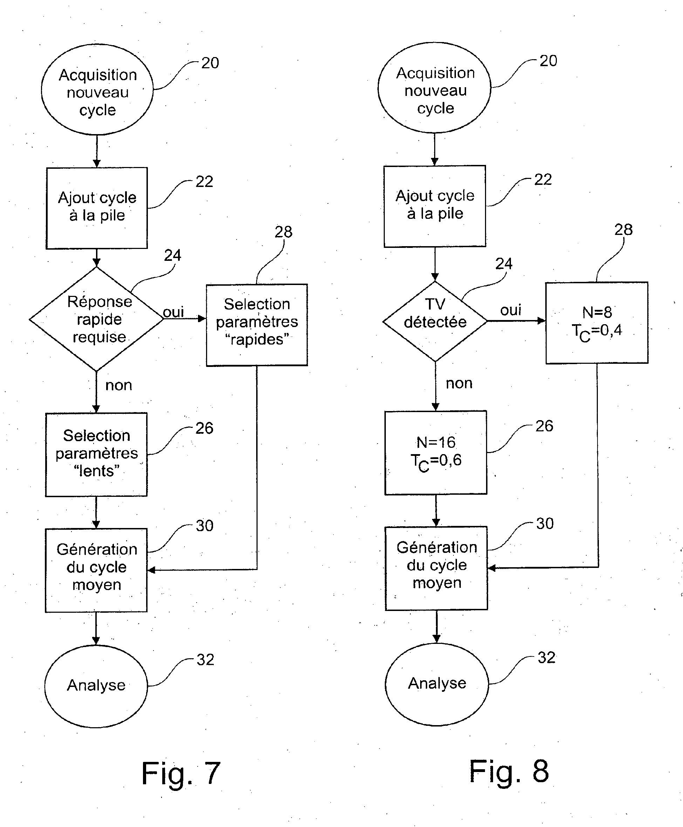

La Figure 7 illustre le principe général de l'ajustement dynamique du traitement selon l'invention.

À chaque nouveau cycle de signal EA acquis et individualisé de la manière exposée plus haut (bloc 20), ce cycle est ajouté à la pile de valeurs échantillonnées constituée avec les N cycles précédents (bloc 22).

Le procédé analyse alors le type de réponse requis (bloc 24). Cette interrogation peut être réalisée en allant chercher dans la mémoire de l'implant les divers indicateurs produits par l'analyse du rythme du patient (par exemple un indicateur d'apparition d'arythmie), de l'état du patient (par exemple un indicateur de réveil), etc.

Si aucune réponse rapide n'est requise, le calcul du signal EA global moyen s'effectue sur la base de paramètres et de critères de validation "lents" (bloc 26). Dans le cas contraire, les paramètres et critères de validation "rapides" sont sélectionnés (bloc 28).

Le cycle moyen est alors généré à partir des paramètres et critères de validation ainsi sélectionnés (bloc 30), pour donner un signal EA global moyen avec, selon le cas, un temps de réponse rapide ou lent, et une qualité de signal plus ou moins grande, ce signal prétraité étant ensuite soumis à une analyse proprement dite (bloc 32).

La Figure 8 illustre un exemple particulier de cette technique.

Dans ce cas, le critère de choix entre paramètres lents ou rapides est la transition d'un rythme sinusal à une tachycardie ventriculaire (TV).

En cas de rythme sinusal, on sait que le signal EA est relativement stable et varie peu d'un cycle à l'autre ; un temps de réponse rapide n'est pas nécessaire et un signal moyen plus précis pourra être obtenu. On peut alors choisir un seuil minimal $T_c$ pour le coefficient de corrélation relativement élevé, par exemple $T_c$ = 0,6, et opérer un moyennage sur un nombre de cycles N relativement élevé, par exemple N = 16 (bloc 26).

En revanche, en cas de détection d'une TV, il convient de disposer d'une réponse rapide du cycle moyen, et l'on sait d'autre part que la variabilité cycle à cycle du signal EA peut être importante. On peut alors réduire par exemple à $N$ = 8 cycles le nombre de cycles utilisés pour le moyennage du signal - ce qui divise par deux le temps de réponse -, et réduire le seuil de coefficient de corrélation à $T_c$ = 0,4 par exemple. De cette manière, le dispositif pourra ainsi rapidement évaluer la possibilité pour le patient de tolérer la TV et d'appliquer rapidement, si besoin, une thérapie appropriée.

**Revendications**

1. Un dispositif d'analyse d'un signal EA d'accélération endocardiaque, comprenant des moyens de traitement du signal EA avec :

   - des moyens de recueil en continu d'un signal EA sur une séquence de *N* cycles cardiaques ;
   - des moyens (10) de découpage du signal EA recueilli, en sous-signaux EA chacun sur la durée d'un cycle cardiaque ;
   - des moyens séparateurs (12), aptes à isoler dans chacun des sous-signaux EA, sur la durée d'une fenêtre d'analyse respective, au moins une composante EAx associée à l'un des bruits cardiaques S1, S2, S3 ou S4 pour ce sous-signal ;

- des moyens (12, 14, 14') pour opérer une intercorrélation entre les sous-signaux EA de ladite au moins une composante EAx, aptes à :

• définir un cycle de référence,
• déterminer pour chaque cycle le coefficient de corrélation est le décalage temporel de la composante EAx par rapport au cycle de référence, et
• opérer un recalage temporel relatif de la composante EAx par rapport au cycle de référence ;

- des moyens (12) de sélection, aptes à appliquer aux sous-signaux EA et aux composantes EAx associées de la séquence de $N$ cycles cardiaques une série de critères de validation et ne retenir que les $N'$ cycles répondant à ces critères, avec $N' \leq N$ ;
- des moyens (16) de moyennage des $N'$ cycles cardiaques, aptes à délivrer en sortie un signal EA global moyen sur un cycle ;
- des moyens de détection (24), aptes à détecter un changement d'état du patient ou la survenue d'un événement prédéterminé chez le patient ; et
- des moyens (26, 28) d'adaptation dynamique des moyens de traitement, aptes à modifier, sur détection dudit changement d'état ou dudit événement prédéterminé, au moins l'un desdits critères de validation et/ou au moins l'un des paramètres des moyens de traitement,

**caractérisé en ce que** les moyens d'adaptation dynamique des moyens de traitement sont des moyens aptes à modifier au moins l'un des paramètres suivants:

• nombre $N$ de cycles de la séquence recueillie et traitée ; et
• pas séparant deux séquences recueillies et traitées successives,

et **en ce que** les moyens d'adaptation dynamique des moyens de traitement sont des moyens aptes à modifier au moins l'un des critères de validation suivants :

• seuil minimal pour ledit coefficient de corrélation par rapport au cycle de référence ;
• seuil maximal pour ledit décalage temporel par rapport au cycle de référence ;
• nombre minimal de cycles pour les $N'$ cycles retenus par les moyens de sélection ; et
• en cas de pluralité de composantes EAx isolées dans chacun des sous-signaux EA, sélection des seuls sous-signaux EA dont toutes les composantes EAx associées répondent aux critères de validation.

2. Le dispositif de la revendication 1, dans lequel ledit changement d'état ou événement prédéterminé est choisi dans le groupe formé par : augmentation du rythme cardiaque, instabilité du rythme cardiaque, diminution de l'amplitude du pic d'accélération endocardiaque, augmentation du rythme ventilatoire, détection d'apnée.

**Patentansprüche**

1. Vorrichtung zur Analyse eines endokardialen Beschleunigungssignals EA, umfassend Mittel zur Verarbeitung des Signals EA mit:

- Mitteln für den kontinuierlichen Empfang eines Signals EA auf einer Sequenz von N Herzzyklen;
- Mittel (10) zum Schneiden des empfangenen Signals EA in Untersignale EA jeweils über die Dauer eines Herzzyklus;
- Trennmitteln (12), die geeignet sind, in jedem der Untersignale EA über die Dauer eines jeweiligen Analysefensters mindestens eine Komponente EAx zu isolieren, die einem der Herzgeräusche S1, S2, S3 oder S4 für dieses Untersignal zugeordnet ist;
- Mitteln (12, 14, 14'), um eine Interkorrelation zwischen den Untersignalen EA der mindestens einen Komponente EAx durchzuführen, die geeignet sind:

• einen Referenzzyklus zu definieren,
• für jeden Zyklus den Korrelationskoeffizienten und den Zeitversatz der Komponente EAx in Bezug zum Referenzzyklus zu bestimmen, und
• eine Zeitnachstellung im Zusammenhang mit der Komponente EAx in Bezug zum Referenzzyklus durchzuführen;

- Auswahlmitteln (12), die geeignet sind, an die Untersignale EA und die Komponenten EAx, die der Sequenz von N Herzzyklen zugeordnet sind, eine Reihe von Validierungskriterien anzulegen und nur die N' Zyklen zu berücksichtigen, die diesen Kriterien entsprechen, wobei N' ≤ N;
- Mitteln (16) zur Mittelwertbildung der N' Herzzyklen, die geeignet sind, am Ausgang ein mittleres globales Signal EA über einen Zyklus zu liefern;
- Erfassungsmitteln (24), die geeignet sind, eine Zustandsänderung des Patienten oder das Eintreten eines vorbestimmten Ereignisses bei dem Patienten zu erfassen; und
- Mitteln (26, 28) zur dynamischen Anpassung der Verarbeitungsmittel, die geeignet sind, bei Erfassung der Zustandsänderung oder des vorbestimmten Ereignisses mindestens eines der Validierungskriterien und/oder mindestens einen der Parameter der Verarbeitungsmittel zu ändern,
**dadurch gekennzeichnet, dass** die Mittel zur dynamischen Anpassung der Verarbeitungsmittel Mittel sind, die geeignet sind, mindestens einen der folgenden Parameter zu ändern:

• Anzahl N von Zyklen der empfangenen und verarbeiteten Sequenz; und
• Abstand zwischen zwei aufeinanderfolgenden empfangenen und verarbeiteten Sequenzen,

und dass die Mittel zur dynamischen Anpassung der Verarbeitungsmittel Mittel sind, die geeignet sind, mindestens eines der folgenden Validierungskriterien zu ändern:

• Mindestschwelle für den Korrelationskoeffizienten in Bezug zum Referenzzyklus;
• Höchstschwelle für den Zeitversatz in Bezug zum Referenzzyklus;
• Mindestanzahl von Zyklen für die N' Zyklen, die von den Auswahlmitteln berücksichtigt werden; und
• im Falle einer Vielzahl von isolierten Komponenten EAx in jedem der Untersignale EA Auswahl nur der Untersignale EA, bei denen alle zugehörigen Komponenten EAx den Validierungskriterien entsprechen.

2. Vorrichtung nach Anspruch 1, der der die Zustandsänderung oder das vorbestimmte Ereignis in folgender Gruppe ausgewählt werden: Erhöhung des Herzrhythmus, Instabilität des Herzrhythmus, Verringerung der Amplitude der endokardialen Beschleunigungsspitze, Erhöhung des Atmungsrhythmus, Erfassung einer Apnoe.


**Claims**

1. A device for analysing an endocardial acceleration, EA, signal, comprising means for processing the EA signal with:

- means for continuously collecting an EA signal over a sequence of N cardiac cycles;
- means (10) for cutting the collected EA signal into EA sub-signals, each over the duration of a cardiac cycle;
- separator means (12), adapted to isolate in each of the EA sub-signals, over the duration of a respective analysis window, at least one EAx component associated with one of the cardiac noises S1, S2, S3 or S4 for this sub-signal;
- means (12, 14, 14') for operating an intercorrelation between the EA sub-signals of said at least one EAx component, adapted to:

. define a reference cycle,
. determine for each cycle the coefficient of correlation and the time offset of the EAx component with respect to the reference cycle, and
. operate a relative time adjustment of the EAx component with respect to the reference cycle;

- selection means (12), adapted to apply to the EA sub-signals and to the associated EAx components of the sequence of N cardiac cycles a series of validation criteria and to retain only the N' cycles fulfilling these criteria, with $N' \leq N$;
- means (16) for averaging the N' cardiac cycles, adapted to deliver as an output a global mean EA signal over one cycle;
- detection means (24), adapted to detect a change of state of the patient or the occurrence of a predetermined event in the patient; and
- means (26, 28) for the dynamic adaptation of the processing means, adapted to modify, over a detection of said change of state or said predetermined event, at least one of said validation criteria and/or at least one of the parameters of the processing means,

**characterized in that** the means for the dynamic adaptation of the processing means are means adapted to modify at least one of the following parameters:

. number $N$ of cycles of the sequence collected and processed; and
. pitch separating two successive sequences collected and processed,

and **in that** the means for the dynamic adaptation of the processing means are means adapted to modify at least one of the following validation criteria:

. minimum threshold for said coefficient of correlation with respect to the reference cycle;
. maximum threshold for said time offset with respect to the reference cycle;
. minimum number of cycles for the N' cycles retained by the selection means; and
. in case of a plurality of EAx components isolated in each of the EA sub-signals, selecting the only EA sub-signals all the associated EAx components of which fulfil the validation criteria.

2. The device of claim 1, wherein said change of state or predetermined even is chosen in the group formed by: cardiac rhythm increase, cardiac rhythm instability, endocardial acceleration peak amplitude decrease, ventilatory rhythm increase, apnea detection.

**Fig. 1**

**Fig. 2**

DÉCOUPAGE SIGNAL EN CYCLES CARDIAQUES — 10

SÉLECTION DES COMPOSANTES EA1 ET EA2
SATIFAISANT LES CONDITIONS DE CORRÉLATION — 12

RECALAGE TEMPOREL
DES NCYCLES EA1   14

RECALAGE TEMPOREL
DES NCYCLES EA2   14'

GÉNÉRATION DU CYCLE MOYEN — 16

**Fig. 3**

Fig. 4

Fig. 5

Fig. 6

Fig. 7

Fig. 8

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2092885 A1
- EP 0515319 A1